# EUROPEAN PATENT APPLICATION

(11) **EP 4 480 951 A1**
(43) Date of publication of application: **25.12.2024**
(21) Application number: 23181049.0
(22) Date of filing: 22.06.2023
(51) Int. Cl.: C07D 413/12

(54) **PROCESS FOR THE PREPARATION OF PYROXASULFONE**

(71) Applicant: Adama Agan Ltd., 7710001 Ashdod (IL)
(72) Inventor: COHEN, Yair, 7685925 Mazkeret Batya (IL); FRONTON, Sveta, 7174650 Modiin (IL); ASHUSH, Natali, 4284200 Bat Hefer (IL)
(74) Representative: Modiano, Gabriella Diana

(57) **Abstract**

The present application relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, wherein the solvent comprises a carbonate.

## Description

### FIELD OF THE INVENTION

The present invention relates to the field of synthesis of organic compounds, more specifically to a process for the preparation of pyroxasulfone.

### BACKGROUND PRIOR ART

US2023012374 discloses the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) by oxidizing 3-[(5-difluoromethoxy-1-methyl-3-trifluoromethylpyrazol-4-yl)methylthio]-4,5-dihydro-5,5-dimethylisoxazole in the presence of a metal catalyst and hydrogen peroxide. The yields of the reaction are typically lowered by incomplete oxidation of the thio group to the corresponding sulfoxide. Different solvents are tested in US2023012374, for example, alcohols, nitriles (e.g. acetonitrile) or carboxylic acid esters, showing different degrees of reduction of the sulfoxide intermediate impurity.

### SUMMARY OF THE INVENTION

The inventors have now realized that the oxidation in the presence of a carbonate ester as solvent greatly reduces the sulfoxide impurity.

Thus, the present application is directed to a process for the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, wherein the solvent comprises a C₁-C₂₄ carbonate ester.

The reaction can be summarized in the following equation:

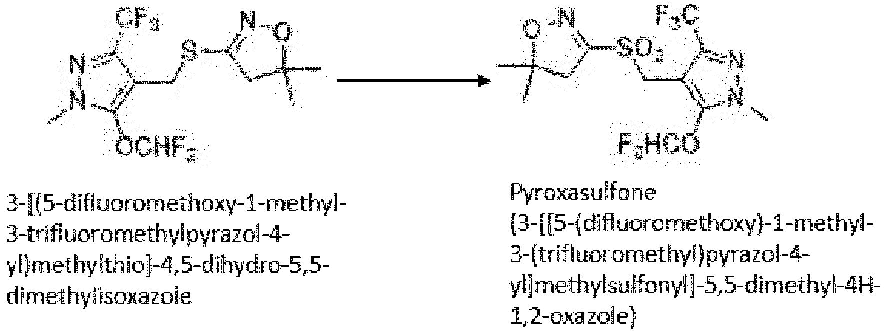

### Definitions

The term "C₁-C₁₂ alkyl" means a linear or branched saturated hydrocarbon chain radical having no multiple bonds and having from one to twelve carbon atoms, and which is attached to the rest of the molecule by a single bond. Suitable groups include, but are not limited to alkyl groups such as methyl, ethyl, propyl (e.g. *n*-propyl or *iso*-propyl), butyl (e.g. *n*-butyl, *t*-butyl, *sec*-butyl), pentyl (e.g. 1-methylpentyl, 3-methylpentyl, *n*-pentyl), hexyl, octyl, or dodecyl.

The term "C₂-C₁₂ alkenyl" means a linear or branched hydrocarbon chain radical having one or more carbon-carbon double bonds therein and having from two to twelve carbon atoms, and which is attached to the rest of the molecule by a single bond. The double bond of an alkenyl group can be unconjugated or conjugated to another unsaturated group. Suitable alkenyl groups include, but are not limited to alkenyl groups such as vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl,), 2-propyl-2-butenyl, 4,6-Dimethyl-oct-6-enyl.

The term "C₂-C₁₂ alkynyl" means a linear or branched hydrocarbon chain radical having one or more carbon-carbon triple bonds therein and from two to twelve carbon atoms, and which is attached to the rest of the molecule by a single bond. The triple bond of an alkynyl group can be unconjugated or conjugated to another unsaturated group. Suitable alkynyl groups include, but are not limited to alkynyl groups such as ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl,), hexynyl (e.g. 1-hexynyl, 2-hexynyl, 3-hexynyl, 4-hexynyl, 5-hexynyl,), methylpropynyl, 3-methyl-1-butynyl, 4-methyl-2-heptynyl, and 4-ethyl-2-octynyl.

"C₆-C₁₅ Aryl" refers to a hydrocarbon moiety having six to fifteen carbon atoms and at least one aromatic hydrocarbon structure, such as phenyl, naphthyl or anthracyl.

"C₇-C₁₅ Arylkyl" refers to an aryl group linked to the rest of the molecule by an alkyl group, such as benzyl and phenethyl.

In each case, the groups "C₁-C₁₂ alkylene", "C₁-C₁₂ alkenylene", "C₁-C₁₂ alkynylene", "C₆-C₁₅ arylene", and "C₇-C₁₅ arylalkylene" have the same meaning as "C₁-C₁₂ alkyl", "C₁-C₁₂ alkenyl", "C₁-C₁₂ alkynyl", "C₆-C₁₅ aryl", and "C₇-C₁₅ arylalkyl", respectively, but wherein the groups are attached to the rest of the molecule by two single bonds. For example, where ethyl represent "CH₃-CH₂-", ethylene represents -CH₂-CH₂-, or where propyl represents CH₃-CH₂-CH₂-, propylene represents - CH₂-CH₂-CH₂- or -CH₂-CH(CH₃)-.

### Examples

### Dimethyl carbonate (PMC)

3-[(5-difluoromethoxy-1-methyl-3-trifluoromethylpyrazol-4-yl)methylthio]-4,5-dihydro-5,5-dimethylisoxazole (100 gr, 78 %) is added to a 1-liter reactor followed by 200 ml of dimethyl carbonate (DMC) and stirring is started. The mixture is heated to 55 °C. A solution of catalyst is prepared as follows: Sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the begging of the addition a slight exotherm is observed, and after it's detraction the temperature is increased to 90 °C. After the addition of hydrogen peroxide, the second half of the catalyst solution is added. After aging of additional 4 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.3% (230 nm). Then 100ml of MeOH are added followed by the addition of 100ml of water. The mixture is heated to 82-85 °C for 1 hour. Then the mixture is cooled to 15°C along 2 hours. The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 90.87%

### Diethyl carbonate (DEC)

3-[(5-difluoromethoxy-1-methyl-3-trifluoromethylpyrazol-4-yl)methylthio]-4,5-dihydro-5,5-dimethylisoxazole (100 gr, 78 %) is added to a 1-liter reactor followed by 100 ml of diethyl carbonate (DEC) and stirring is started. The mixture is heated to 55 °C. A solution of catalyst is prepared as follows: sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the begging of the addition a slight exotherm is observed, and after it's detraction the temperature is increased to 90 °C. After the addition of hydrogen peroxide, the second half of the catalyst solution is added. After aging of additional 6 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.31% (230 nm). Then 100ml of MeOH are added followed by the addition of 100ml of water. The mixture is heated to 82-85 °C for 1 hour. Then the mixture is cooled to 15°C along 2 hours. The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 87.49%

### Propylene carbonate (PC)

3-[(5-difluoromethoxy-1-methyl-3-trifluoromethylpyrazol-4-yl)methylthio]-4,5-dihydro-5,5-dimethylisoxazole (100 gr, ~78 %) is added to a 1-liter reactor followed by 100 ml of propylene carbonate (PC) and stirring is started. The mixture is heated to 55 °C. A solution of catalyst is prepared as follows: sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the begging of the addition a slight exotherm is observed, and after it's detraction the temperature is increased to 90 °C. After the addition of hydrogen peroxide, the second half of the catalyst solution is added. After aging of additional 4 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.05% (230 nm). Then 100ml of MeOH are added followed by the addition of 100ml of water. The mixture is heated to 82-85 °C for 1 hour. Then the mixture is cooled to 15°C along 2 hours. The mixture was filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 88.48%

### Ethylene carbonate (EC)

3-[(5-difluoromethoxy-1-methyl-3-trifluoromethylpyrazol-4-yl)methylthio]-4,5-dihydro-5,5-dimethylisoxazole (100 gr, 78 %) is added to a 1-liter reactor followed by 100 ml of Ethylene carbonate (EC) and stirring is started. The mixture is heated to 55 °C. A solution of catalyst is prepared as follows: sodium tungstate hydrate (0.02 eq. 1.42 gr) is dissolved in 10 ml of water. Half of the catalyst solution (5.7 gr) is added to the reactor. Then, the reaction is fed with hydrogen peroxide (28-30%, 4.2 eq., 103.36 gr) during 2 hrs. At the begging of the addition a slight exotherm is observed, and after it's detraction the temperature is increased to 90 °C. After the addition of hydrogen peroxide, the second half of the catalyst solution is added. After aging of additional 2 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 0.19% (230 nm). Then 100ml of MeOH are added followed by the addition of 100ml of water. The mixture is heated to 82-85 °C for 1 hour. Then the mixture is cooled to 15°C along 2 hours. The mixture is filtered, the resulting crystals are washed with cold MeOH (2 X 100ml), NaHCO3 (5%, 100 ml) and water (2 X 100ml), and dried over night at 50 °C under vacuum, to obtain pyroxasulfone with the yield of 84%.

## Claims

1. A process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, wherein the solvent comprises a C₁-C₂₄ carbonate ester.

2. The process according to claim 1, wherein the carbonate is a compound of formula (I) 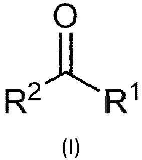 wherein
each of R¹ and R² is selected from the group consisting of C₁-C₁₂ alkyl, C₁-C₁₂ alkenyl, C₁-C₁₂ alkynyl, C₆-C₁₅ aryl, and C₇-C₁₅ arylalkyl; or
both R¹ and R² together form a group selected from C₁-C₁₂ alkylene, C₁-C₁₂ alkenylene, C₁-C₁₂ alkynylene, C₆-C₁₅ arylene, and C₇-C₁₅ arylalkylene.

3. The process according to claim 2, wherein the carbonate each of R¹ and R² is selected from the group consisting of C₁-C₁₂ alkyl.

4. The process according to any of claims 2 or 3, wherein the carbonate each of R¹ and R² is selected from the group consisting of C₁-C₄ alkyl.

5. The process according to any of claims 2 to 4, wherein R¹ and R² are both methyl of ethyl.

6. The process according to claim 2, wherein R¹ and R² together are a C₁-C₁₂ alkylene.

7. The process according to any of claims 2 or 6, wherein R¹ and R² together are a C₁-C₄ alkylene.

8. The process according to any of claims 2, 6 or 7, wherein R¹ and R² together are -(CH₂-CH₂)- or -(CH(CH)₃)CH₂)-.

9. The process according to any of the previous claims, wherein the solvent comprises a C₁-C₂₄ carbonate ester and water.

10. The process according to any of the previous claims, wherein the hydrogen peroxide is a 10 to 70 wt percent aqueous hydrogen peroxide solution.

11. The process according to any of the previous claims, wherein the metal catalyst is selected from a tungsten catalyst, a molybdenum catalyst and a niobium catalyst.

12. The process according to claim 11, wherein the metal catalyst is a tungsten catalyst.

13. The process according to claim 12, wherein the metal catalyst is selected from the group consisting of tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.

14. The process according to claim 13, wherein the metal catalyst is sodium tungstate.

15. The process according to any of the previous claims, wherein the temperature of the reaction is between 30°C and 120°C, preferably between 40°C and 100°C.
